(19) Europäisches Patentamt
European Patent Office
Office européen des brevets

(11) EP 0 501 849 B1

(12) **FASCICULE DE BREVET EUROPEEN**

(45) Date de publication et mention de la délivrance du brevet: **24.01.1996 Bulletin 1996/04**

(51) Int. Cl.[6]: **C07C 43/225**, C09K 19/30, C07C 69/63

(21) Numéro de dépôt: **92400422.9**

(22) Date de dépôt: **18.02.1992**

(54) **Cristaux liquides fluorés**

Fluorierte Flüssigkristalle

Fluorinated liquid crystals

(84) Etats contractants désignés:
**DE GB IT**

(30) Priorité: **26.02.1991 FR 9102257**

(43) Date de publication de la demande:
**02.09.1992 Bulletin 1992/36**

(73) Titulaire: **THOMSON-CSF**
**F-75008 Paris (FR)**

(72) Inventeurs:
- **Vergnolle, Marie**
  **F-92045 Paris la Défense (FR)**
- **Soyer, Françoise**
  **F-92045 Paris la Défense (FR)**
- **Le Barny, Pierre**
  **F-92045 Paris la Défense (FR)**
- **Dubois, Jean-Claude**
  **F-92045 Paris la Défense (FR)**

(74) Mandataire: **Guérin, Michel et al**
**F-92402 Courbevoie Cédex (FR)**

(56) Documents cités:
**EP-A- 0 294 852**
**EP-A- 0 343 487**
**EP-A- 0 350 936**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.9.7/3.4

## Description

La présente invention a pour objet des cristaux liquides chiraux fluorés incorportant un groupement cyclohexyl dans leur structure, utilisables comme dopants et conduisant à des mélanges de cristaux liquides ferroélectriques ayant une application dans les systèmes de visualisation.

Actuellement, seuls les cristaux liquides nématiques ont reçu une application dans le domaine de la visualisation ce qui a conduit principalement aux afficheurs utilisant le mode nématique en hélice et plus récemment aux afficheurs nématiques super torsadés (STN).

L'inconvénient de ces types d'afficheurs est leur mauvais angle de vue et leur temps de réponse élevé, de l'ordre de la dizaine de millisecondes, ce qui limite leur développement.

L'invention en 1980 par N.A. Clark et S. Lagerwall (Applied Physic Letter, Vol. 36, n° 89, 1980) d'un système de visualisation basé sur l'utilisation de cristaux liquides ferroélectriques a conduit à un nouvel effet électrooptique, induit par un couplage linéaire entre la polarisation électrique permanente des molécules et le champ électrique d'adressage ayant des temps de commutation de 3 ordres de grandeur inférieurs à ceux de l'affichage nématique en hélice traditionnel.

Pour des raisons de symétrie, la ferroélectricité de la phase $S_C^*$ ne peut être observée que si la structure hélicoïdale est déroulée. Cette condition est réalisée dans le système de visualisation proposé par Clark et Lagerwall lorsque la phase $S_C^*$ est introduite dans une cellule dont l'épaisseur est inférieure à 2 µm.

Les performances électrooptiques de tels afficheurs dépendent en grande partie du matériau mésomorphe utilisé.

En première approximation, le temps de réponse $\tau$ d'un mélange $S_C^*$ est donné par la relation suivante :

$$\tau = \frac{\eta}{P_S \times E}$$

où $\eta$ représente la viscosité rotationnelle du cristal liquide $P_S$ sa polarisation spontanée et E le champ électrique appliqué.

Les mélanges $S_C^*$ doivent présenter un certain nombre de propriétés comme :

. être stable thermiquement et photochimiquement
. s'aligner facilement
. présenter la succession de phase suivante :
  $S_C^*$, $S_A$, N*, L
. posséder une large gamme en température de mésomorphie
. avoir un angle d'inclinaison voisin de 22,5 °.

Pour obtenir un mélange $S_C^*$ qui remplit toutes ces conditions> on préfère actuellement doper un mélange $S_C$ avec un ou plusieurs dopants.

Le mélange $S_C$ apporte :

. la succession des phases
. le domaine d'existence en température de ces phases
. la faible viscosité
. l'anisotropie diélectrique et la biréfringence.

Les dopants chiraux apportent la chiralité et la polarisation. Le dopant doit par ailleurs ne pas modifier les températures de transition de la matrice $S_C$ et ne pas augmenter la visosité du mélange final.

Selon l'art antérieur, il existe des molécules chirales de formule chimique proche de celles de l'invention mais n'offrant pas les mêmes performances au niveau de la polarisation spontanée de mélanges obtenus avec de telles molécules.

Il peut s'agir de molécules de type :

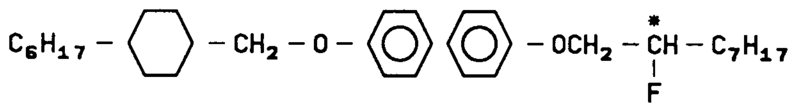

EP-A-0 294 852
ou de molécules de type

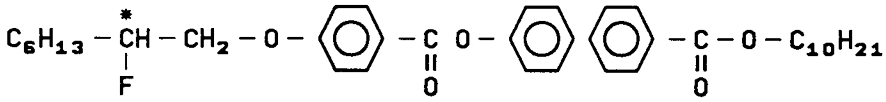

EP-A-0 350 936

Afin de satisfaire toutes ces conditions, la présente invention propose une nouvelle formule de cristaux liquides chiraux fluorés, incorporant un groupement cyclohexyi dans leur structure, utilisables comme dopant d'une matrice $S_C$.

L'invention a donc pour objet une famille de cristaux liquides chiraux fluorés incorporant un groupement cyclohexyl dans leur structure, caractérisée en ce qu'elle répond à la formule générale suivante :

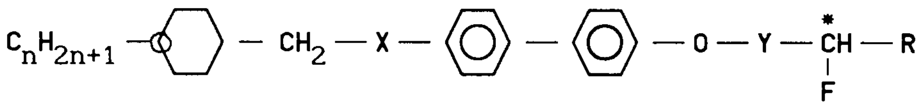

avec $1 \leq n \leq 14$
si $X = CH_2$
$Y = -CH_2-$ avec $R = CmH_{2m+1}$ et $5 \leq m \leq 16$
ou

Y = 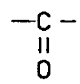

avec

R = 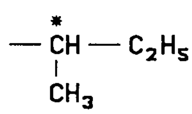 ou 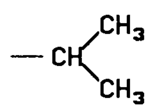

ou $-C_4H_9$
si $X = -O-$

Y = 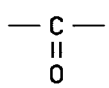

avec

R = 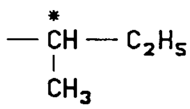 ou 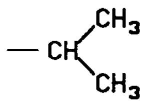

ou $—C_4H_9$

Cette famille présente les avantages suivants :

- la présence de 3 cycles dans la structure chimique des molécules de la formule conduit à l'existence de mésomorphes sur une large gamme de température.
- la liaison C-F qui assure la contribution la plus importante à la polarisation du mélange conduit à des dérivés plus stables que les dérivés chlorés analogues
- enfin l'utilisation d'un radical cyclohexane éthyl trans ou cyclohexylmethoxy concourent à l'obtention de mélanges de faible viscosité comparés aux dérivés benzoïques correspondants.

L'invention sera mieux comprise à la lecture de la description suivante et des dessins annexés parmi lesquels :

- la figure 1 représente le schéma de synthèse d'un premier exemple de molécule selon l'art connu,
- la figure 2 représente le schéma de synthèse d'un exemple de molécule selon l'invention,
- la figure 3 représente l'évolution du temps de réponse électrooptique avec la température pour des mélanges obtenus avec les composés (I) et (II).
- la figure 4 représente l'évolution de la polarisation spontanée (avec la température) de mélanges obtenus avec les composés (I) et (II) ;
- la figure 5 représente l'évolution de l'angle de tilt en fonction de la température pour des mélanges obtenus avec les composés (I) et (II) ;

<u>Synthèse des composés</u> :

* <u>Synthèse du 4-[trans-(4 alkyl cyclohexane) methoxy] 4' (2 fluoro alkyl 1-methoxy) biphényl.</u> (composé I, selon l'art connu)

La synthèse de ces composés se fait en 7 étapes à partir de l'acide 4 alkylbenzoïque et l'époxyde chiral correspondant selon le schéma réactionnel décrit en figure 1. L'acide 4 pentyl cyclohexyl est commercial. L'hydrogénation du noyau benzénique de l'acide 4 alkylbenzoïque et la séparation des isomères cis et trans au moyen d'un composé d'inclusion avec la thiourée ne pose aucun problème à l'homme de l'art. Dans ce qui suit, nous décrirons plus en détail les étapes plus difficiles.

La structure des produits obtenus a été établie par RMN $^1H$ sur spectromètre VARIAN T 60 (et T 80) et BRUKER AM 250 et WPSY 80 en utilisant le tétraméthylsilane (TMS) comme référence interne et le chloroforme deuteré ($CDCl_3$) comme solvant. Les spectres de masse ont été effectués sur un spectromètre quadripole NERMAG R 10 10 C, en ionisation chimique (gaz réactant $NH_3$) et par impact électronique. Les mesures des pouvoirs rotatoires $\alpha_D$ ont été effectuées à la raie jaune du sodium sur un polarimètre Perkin-Elmer (solvant benzène).

<u>Synthèse du Trans-(4-pentylcyclhexane) méthanol</u> correspondant à la deuxième étape de la figure 1.

Dans un tricol et sous argon, ou ajoute goutte à goutte l'acide trans-4-pentylcyclohexanoïque (10g soit 50,5 mmoles ) dans 40 ml de tétrahydrofuranne (THF) sec à une solution de $LiAlH_4$ (2,85 g soit 75,5 mmoles) dans 60 ml de THF à 0°C. L'addition terminée, on porte au reflux pendant une heure.

Puis on refroidit à 0°C, on ajoute alors 150 ml d'$H_2O$ et enfin 20 ml de HCL 2N. On extrait alors la phase aqueuse par 2 fois 100 ml d'éther, on rassemble les phases organiques, on sèche, on filtre et l'on évapore le solvant à sec. On obtient alors 7,9 g d'une huile incolore.

<u>Synthèse du trans-4-(pentylcyclohexane) bromométhane</u> (étape $III_1$)

On dissout dans 50 ml de chlorure de méthylène sec le tétrabromométhane (18,7 g soit 56,5 mmole) et l'alcool précédent (alcool 1).

On ajoute alors la triphenylphosphine solide (14,7 g soit 56,5 mmoles) par fractions ; il apparait une coloration jaune de même qu'un échauffement. Puis on ajoute 200 ml d'une solution éther/éther de pétrole 1/1, on filtre le précipité blanc obtenu et on évapore les eaux mères. L'analyse du spectre RMN à 60 MHZ du produit brut montre qu'il demeure de l'oxyde de triphenylphosphine, celui-ci est purifié par filtration sur silice avec comme éluant de l'heptane. On obtient alors 9,8 g d'une huile incolore.

Synthèse du 2 (+) fluoro octane -1-ol correspond à la 5ème étape ($V_1$)

On refroidit dans un bécher en polyéthylène 30 ml d'acide fluorhydrique (HF)/pyridine sous argon. On ajoute alors goutte à goutte l'époxyde (8,4 g soit 65 mmoles) dans 20 ml d'éther. On laisse agiter 2 heures à température ambiante. On ajoute 100 ml d'eau puis on neutralise le milieu réactionnel par une solution de soude 5N. On obtient 8,4 g d'une huile légèrement jaune qui cristallise. Le produit brut est purifié par chromatographie liquide haute pression (HPLC) sur silice. On isole deux fractions possédant un $\alpha_D$ non nul.

Les mesures sont les suivantes :

fraction a $\alpha_D$ = - 8,9 °
fraction b $\alpha_D$ = - 12,5° ($\alpha$ = - 13,1° littérature)
la fraction B correspond à l'alcool chiral attendu (alcool 3).

Synthèse du 2 (-) fluoro-1-octane (4-méthyl phényl sulfonyl) 4 correspondant à la 6ème étape ($VI_1$)

On ajoute goutte à goutte à 0°C le chlorure de tosyle TSCl (2 g soit 10,5 mmoles) dans 100 ml de chloroforme à une solution de l'alcool 3 (1,1 g soit 7,6 mmoles) et de pyridine (1,4 g soit 17,3 mmoles) dans 20 ml de chloroforme ($CHCl_3$). On laisse 2 heures à température ambiante et on contrôle en chromatographie couche mince (CCM) l'évolution de la réaction ; il demeure de l'alcool fluoré ; on rajoute 0,4 équivalent de pyridine et de tosyle. On laisse 24 heures à température ambiante. On ajoute 30 ml d'eau et 30 ml d'éther. On lave par 10 ml d'HCL 2N, on neutralise par 2 fois 10 ml d'une solution saturée de $NaHCO_3$ et on lave avec 10 ml d'eau. On extrait puis on sèche la phase organique sur $MgSO_4$, on filtre et on concentre. Après purification par HPLC sur silice on récupère 1,66 g d'une huile légèrement jaune.

Synthèse du 4-(trans-(4-pentyl cyclohexane) méthoxy, 4′-hydroxybiphenyl 5 (étape $IV_1$)

On porte au reflux le dihydroxy-4, 4′-biphenyl (10 g soit 53,7 mmoles) et le carbonate de potassium (28,6 g soit 0,27 mmoles) dans 400 ml d'acétonitrile pendant 1 heure. On revient à la température ambiante puis on ajoute le dérivé bromé dans 30 ml d'acétonitrile. On porte au reflux. Après 24 heures il y a peu de produit formé. On ajoute alors 100 ml de toluène et l'on porte à nouveau 24 heures à 80°C. Le suivi CCM montre qu'il n'y a pas d'évolution. On ajoute alors 3 fois 3 ml de 1,5 - diazobicyclo (3-4-0) nonène 5 (DBN).

On filtre, on évapore. On reprend par 300 ml de dichlorométhane, on lave par 40 ml d'acide sulfurique 1N, puis 100 ml d'eau, on extrait les phases aqueuses par 2 fols 50 ml de dichlorométhane. On rassemble les phases organiques, on les sèche sur $MgSO_4$ puis on les filtre et on les concentre. On récupère 1,8 g de brut. On purifie par filtration sur silice.

Synthèse du 4-[trans-(4 pentyl cyclohexane) methoxy], 4′(2 fluoro heptane 1 - méthoxy) biphenyl 6 (étape $VII_1$)

On sèche un tricol de 100 ml à la flamme sous argon. On lave par 10 ml de pentane l'hydrure de sodium, ce toujours sous argon, puis on reprend par 20 ml de THF sec et on ajoute goutte à goutte le phénol monoétherifié 5 (1 g soit 2,8 mmoles) dans 40 ml de THF. On porte au reflux une heure. On revient à la température ambiante et on ajoute le dérivé tosylé (0,86 g soit 2,8 mmoles) dans 20 ml de THF. On porte à reflux pendant 5 heures la réaction n'évoluant plus. On hydrolyse par 50 ml de $NH_4Cl$ puis on lave par 50 ml d'eau en contrôlant le pH. On extrait les phases aqueuses par 3 fois 20 ml d'éther, on sèche sur $MgSO_4$, on filtre, on concentre. On récupère alors 1,4 g de produit brut.

Après purification par HPLC on récupère 510 mg d'un solide blanc.

* Synthèse du 4-[trans-(4 alkyl cyclohexyl) ethyl 4′ (2 fluoro alcanoyloxy] biphényle dont le schéma réactionnel est indiqué à la figure 2 (composé II selon l'invention)

La synthèse de ces composés se fait en 8 étapes à partir de l'acide 4 alkylbenzoïque et de l'acide aminé correspondant (leucine, isoleucine, valine) (figure n° 2).

La synthèse du 4 hydroxy 4' alkyltrans cyclohexyléthyl biphényle est décrite dans le Brevet 89 12026 déposé par la Demanderesse, dont le numéro de publication est 2 651 790.

La synthèse de l'acide 2 fluoro 3 méthyl pentanoïque s'est faite selon un mode opératoire décrit par R. Buchecker, S. M. Kelly and J. Fuenfschilling (Liq. Cryst. 8 (2) 217-227 (1990).

Synthèse de l'acide 2 fluoro 3 methyl pentanoïque (étape $VII_2$)

Dans un bécher en téflon, on introduit 5,24 g (0,04 mole) d'isoleucine puis 100 ml de HF pyridine 70 %.

On refroidit à 15°C et on ajoute 44 ml de pyridine distillée, par petites quantités. On observe la formation de fumées blanches.

On refroidit à 0°C et on ajoute 4,1 g (0,06 mole) de nitrite de sodium. On agite en laissant revenir à la température ambiante, puis on continue d'agiter pendant 5 jours.

On coule la solution dans un mélange de 400 ml d'eau et 400 g de glace.

On extrait à l'éther.

Le produit brut (environ 2 g) est purifié par distillation sous vide $E_7$ = 80°C.

On obtient 400 mg d'acide.

Rendement $\rho$ = 7,5 %.

Synthèse du 4-[trans-(4 heptyl cyclohexyl) éthyl 4' (2 fluoro 3 methyl pentanoyloxy) biphenyle (étape $VIII_2$)

Dans un erlenmeyer on introduit 0,23 g (0,6 mmole) de 4-[trans-(4 heptyl cyclohexyl) ethyl 4' hydroxy biphenyl, 0,4 g (3 mmole) d'acide 2 fluoro 3 methyl pentanoïque, 5 ml de chlorure de méthylène, 0,74 g (3,6 mmole) de dicyclohexylcarbodiimide DCCI et 0,148 g (1 mmole) de piperidinopyridine. On agite à température ambiante pendant 36 heures.

Le précipité d'urée est éliminé par filtration. Le filtrat est évaporé à sec.

Le produit brut est purifié par chromatographie sur $SiO_2$ éluant hexane 60 - Toluène 40 suivie d'une recristallisation dans l'hexane. On obtient 0,18 g de produit.

Rendement 60 %.

Propriétés des corps synthétisés

Les composés selon l'invention ont été étudiés par analyse enthalpique différentielle et par microscopie optique. Les résultats sont rassemblés dans le tableau n° 1.

| Index | n | x | R | Y | Propriétés mésomorphes |
|---|---|---|---|---|---|
| (I) | 5 | O | $C_6H_{13}-$ | - O - | $S_3$85,2 $S_B$145 $S_A$175,5L |
| (II) | 7 | $-CH_2-$ | $C_2H_5-\overset{*}{C}H(CH_3)-$ | - CO - | C 56,5 S 137 L |

**TABLEAU 1**

Le composé (I) ne cristallise pas.

Les phases $S_3$ et S sont des phases smectiques très ordonnées.

Les composés (I) et (II) ont été introduits à titre de dopants dans un mélange M présentant une phase $S_C$(mélange Merck référencée ZLI3234B) et possédant la succession de phases suivantes :

$S_C$ 76 $S_A$ 80 N 96 L

Les propriétés suivantes ont été mesurées :

- températures de transition du mélange final,
- polarisation en fonction de la température,
- angle de tilt en fonction de la température,
- mesure du temps de réponse.

Pour obtenir un mélange homogène après incorporation du dopant on a procédé de la manière suivante. Après pesée du dopant et du mélange M dans un même récipient, on réalise une solution homogène par ajout d'un solvant

purifié à bas point d'ébullition ($CH_2Cl_2$ ou $CHCl_3$). Cette solution est ensuite filtrée sur filtre Millipore 1 µm en environnement dépoussiéré. Enfin, le solvant est éliminé par évaporation lente sous courant d'azote à 40°C, puis sous vide.

Tous les dopants selon l'invention sont solubles à une concentration de 10 % en poids dans le mélange.

Les températures de transition des mélanges dopés (à raison de 10 % en poids de dopant dans le mélange final obtenu) ont été déterminées par analyse enthaipique différentielle et par microscopie optique. Les résultats sont rassemblés dans le tableau 2.

TABLEAU 2

| dopant | $S_C^*$ | | $S_A$ | | N* | | L |
|---|---|---|---|---|---|---|---|
| (I) | ∘ | 71,5 | ∘ | 85,5 | ∘ | 103 | ∘ |
| (II) | ∘ | 68,8 | ∘ | 87,8 | ∘ | 98,5 | ∘ |

La mesure de polarisation, du temps de réponse et de l'angle de tilt nécessite la réalisation de cellules de mesures. Les cellules sont faites à partir de lames de verre rectangulaires (2 x 3 cm) de 1 mm d'épaisseur, recouvertes d'ITO (oxyde mixte d'indium et d'étain) non recuit. La fabrication des cellules se fait en 5 étapes.

On définit d'abord sur chaque lame de verre une bande conductrice de 1 cm de largeur.

Puis on enduit ces lames d'un film d'alcool polyvinylique (Rhodoviol 4,125) par dépôt à la tournette d'une solution aqueuse à 3 %. Après séchage à 100°C pendant une heure, le film de PVA est frotté au moyen d'une machine de frottement (par exemple de la marque Asulab) afin d'induire une orientation planaire non dégénérée. Les cellules sont ensuite scellées au moyen d'une col le araldite. Les contacts entre un fil électrique et une lame sont réalisés à l'aide de points de colles à l'argent. Enfin le matériau est introduit par capillarité.

La mesure de polarisation a été faite en utilisant un signal triangulaire. Le champ d'excitation utilisé est de 15 V/µm. Les mesures ont été faites à 100 Hz.

La figure 4 représente la polarisation spontanée des mélanges obtenus avec les composés (I) et (II).

La mesure de l'angle de tilt est faite en utilisant une méthode électrooptique. Un champ continu (+ E) est appliqué aux bornes de la cellule qui est observée au microscope entre polariseur et analyseur croisés. Les dipôles s'alignent dans le sens du champ et on détermine la position d'extinction. Ensuite le sens du champ est inversé (- E), ce qui inverse le sens des dipôles. Les molécules ont alors tourné d'un angle 2 Φ. Il faut tourner l'échantillon d'un angle 2 Φ pour retrouver une position d'extinction. Le sens dans lequel il faut faire tourner l'échantillon pour retrouver une position d'extinction fournit également le signe de la polarisation. Le champ électrique appliqué est de 15 V/µm.

La figure 5 représente l'angle de tilt en fonction de la température des mélanges obtenus avec les composés (I) et (II).

Le temps de réponse électrooptique des matériaux a été mesuré en appliquant une tension en forme de créneaux aux bornes d'une cellule de mesure, correspondant à un champ électrique de 15 V/µm.

La figure 6 représente l'évolution du temps de réponse τ en fonction de la température pour les mélanges obtenus avec les composés (I) et (II).

## Revendications

1. Molécule chirale, cristal liquide caractérisée en ce qu'elle répond à la formule générale suivante :

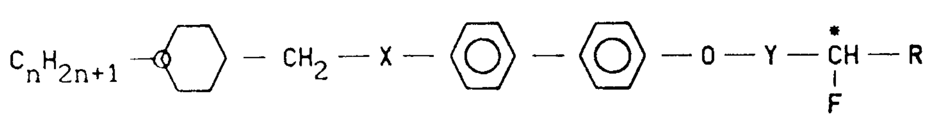

avec $1 \leq n \leq 14$

si X = $CH_2$

Y = —$CH_2$— avec R = $C_mH_{2m+1}$ et $5 \leq m \leq 16$

ou

Y = 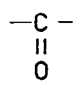

avec

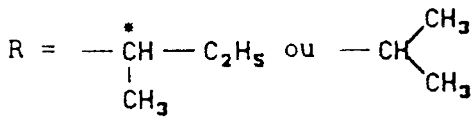

ou $—C_4H_9$
si X = —O—

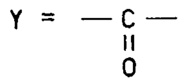

avec

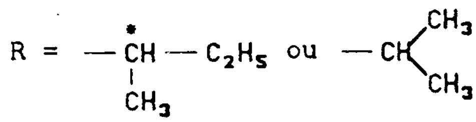

ou $—C_4H_9$

2. Molécule selon la revendication 1, caractérisée en ce que
X = $—CH_2$ et

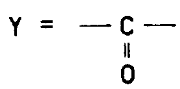

avec

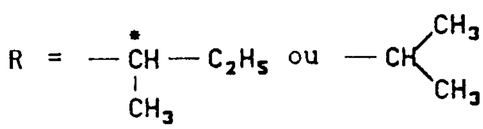

ou $—C_4H_9$

## Claims

1. Chiral liquid-crystal molecule, characterized in that it corresponds to the following general formula:

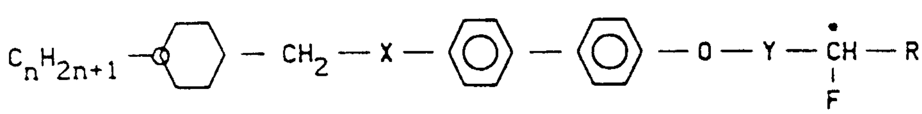

with $1 \leq n \leq 14$
if X = $CH_2$

Y = $-CH_2-$ with R = $C_mH_{2m+1}$ and $5 \leq m \leq 16$ or

Y = 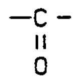

with

R = 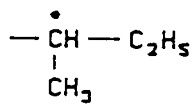 or 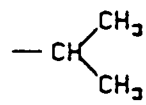

or $-C_4H_9$
if X = —O—

Y = 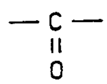

with

R = 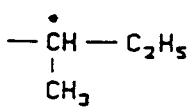 or 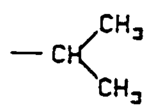

or $-C_4H_9$

**2.** Molecule according to Claim 1, characterized in that X = $-CH_2$ and

Y = 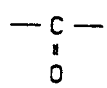

with

R = 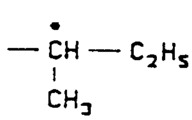 or 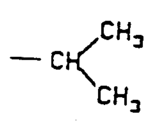

or $-C_4H_9$

## Patentansprüche

1. Chirales Flüssigkristallmolekül, **dadurch gekennzeichnet**, daß es die folgende allgemeine Formel aufweist:

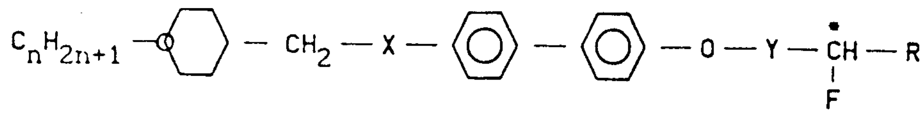

mit $1 \leq n \leq 14$
wenn $X = CH_2$
$Y = -CH_2-$ mit $R = CmH_{2m+1}$ und $5 \leq m \leq 16$
oder

Y = 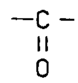

mit

R = 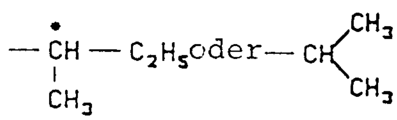

oder $-C_4H_9$
wenn $X = -O-$

Y = 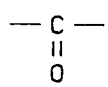

mit

R = 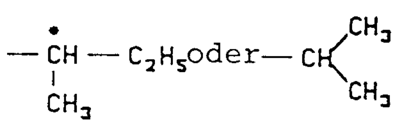

oder $-C_4H_9$

2. Molekül nach Anspruch 1, **dadurch gekennzeichnet**, daß
$X = -CH_2$ und

Y = 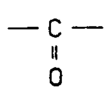

mit

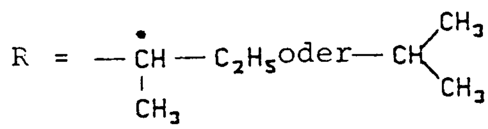

oder $—C_4H_9$

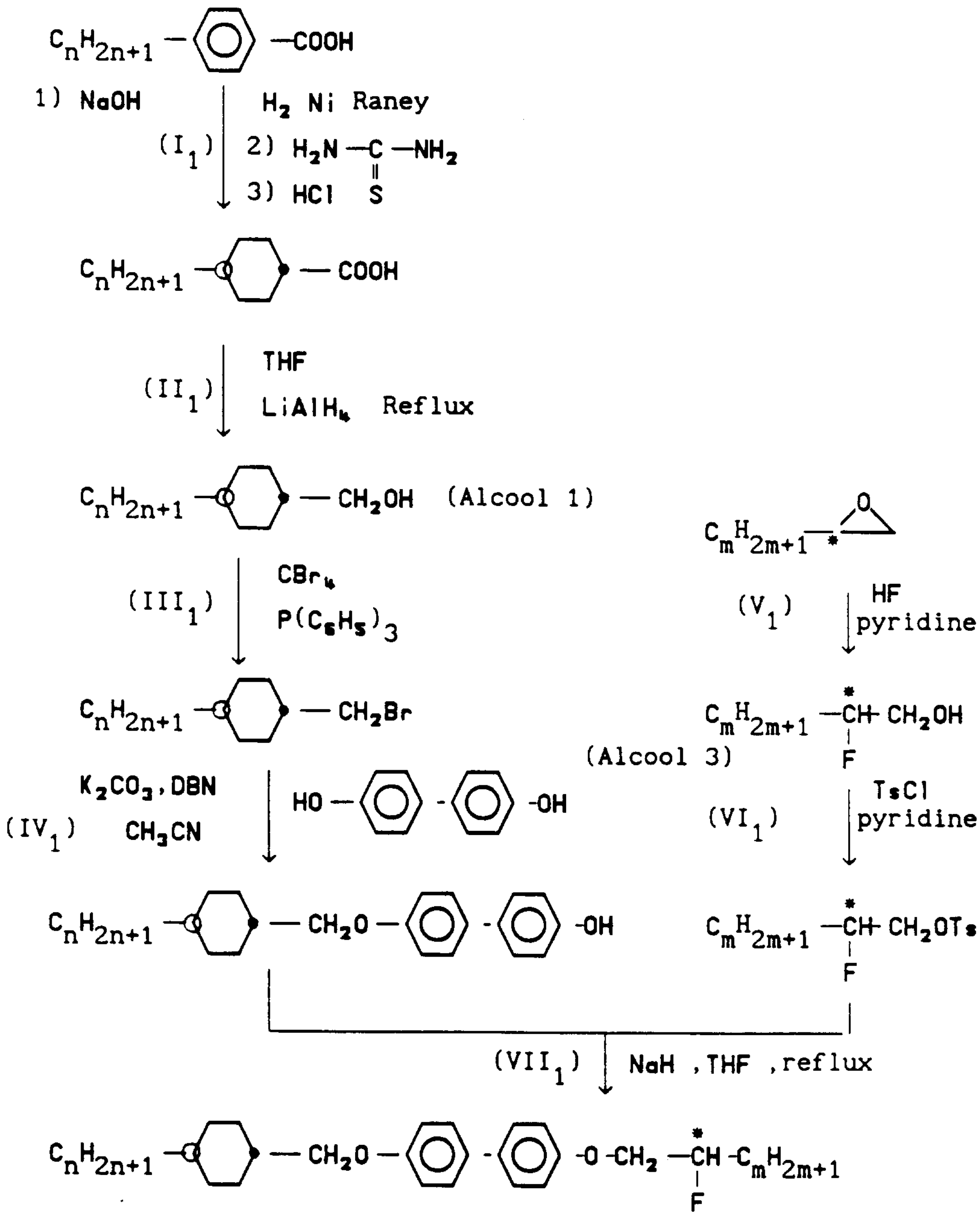
1) NaOH
H2 Ni Raney
(I1)
3) HCl
(II1)
THF
LiAlH4 Reflux
(Alcool 1)
(III1)
CBr4
P(C6H5)3
(V1)
HF
pyridine
(Alcool 3)
(IV1)
K2CO3, DBN
CH3CN
(VI1)
TsCl
pyridine
(VII1)
NaH, THF, reflux

FIGURE I

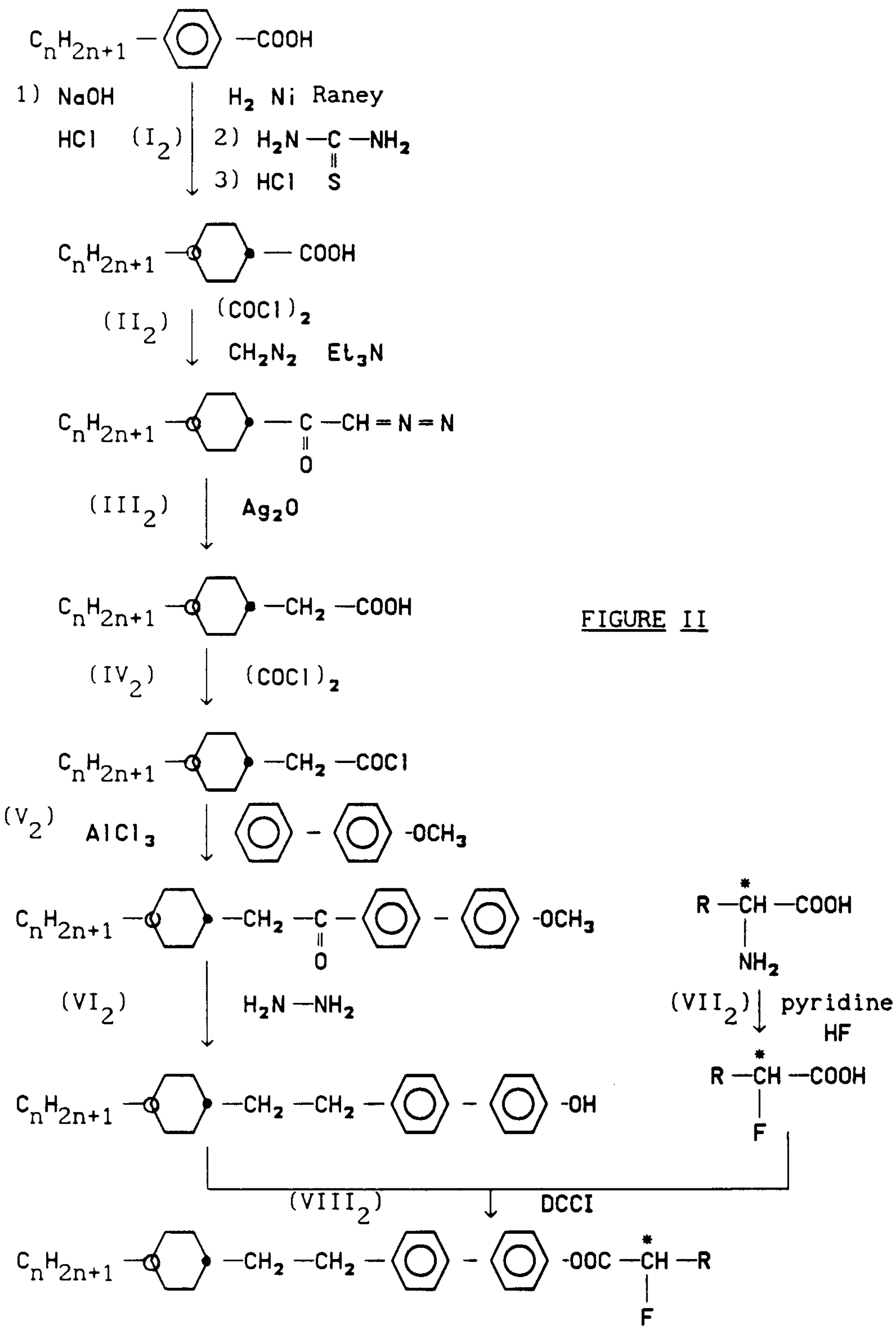
1) NaOH
HCl
($I_2$)
$H_2$ Ni Raney
2) $H_2N-C(=S)-NH_2$
3) HCl
($II_2$)
$(COCl)_2$
$CH_2N_2$ $Et_3N$
($III_2$)
$Ag_2O$
($IV_2$)
$(COCl)_2$
($V_2$)
$AlCl_3$
($VI_2$)
$H_2N-NH_2$
($VII_2$)
pyridine
HF
($VIII_2$)
DCCI

FIGURE II

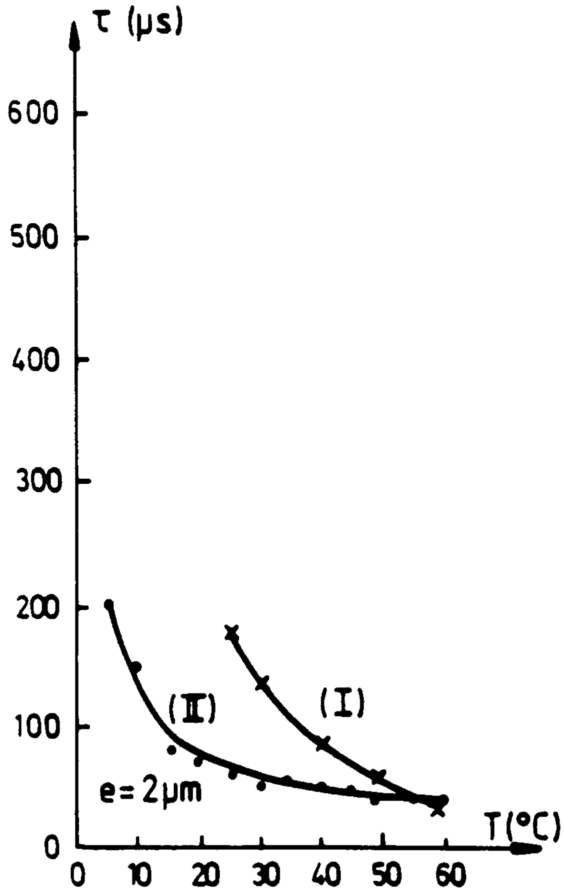


FIG.3

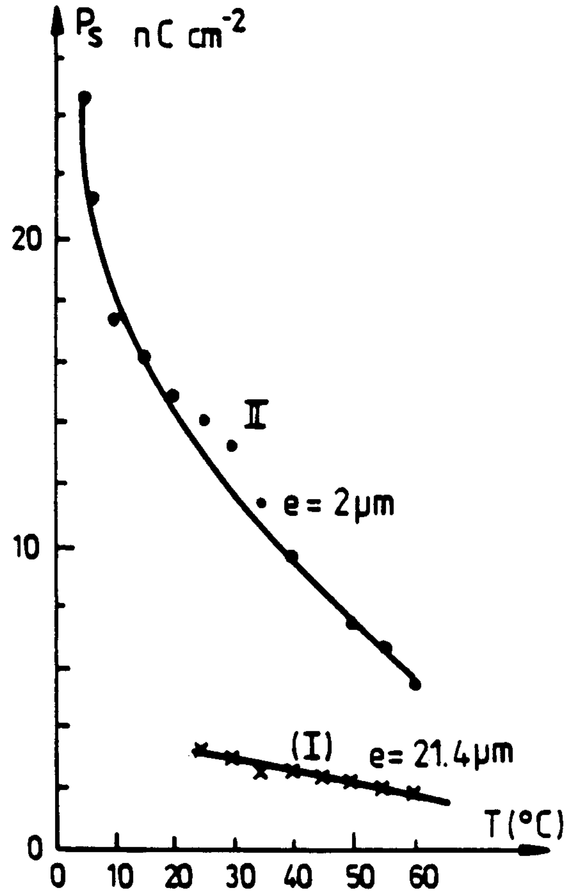


FIG.4

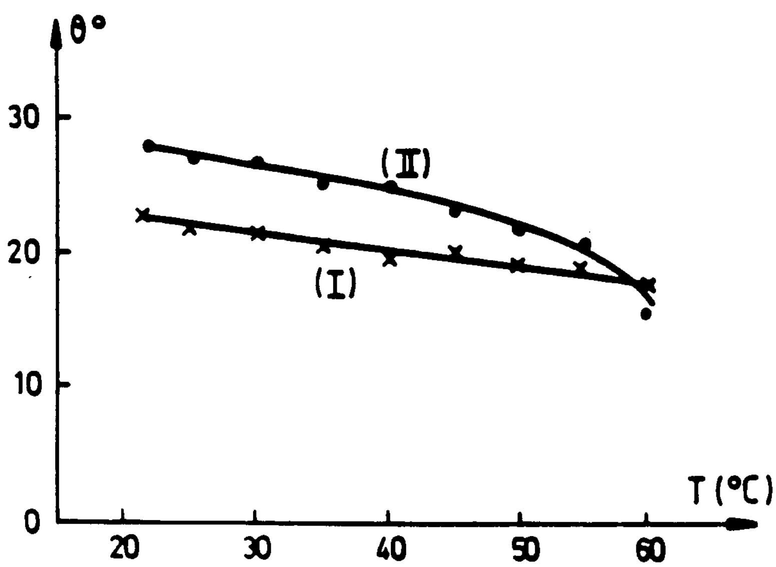


FIG.5